# EUROPEAN PATENT APPLICATION

(11) **EP 2 502 901 A1**
(43) Date of publication of application: **26.09.2012**
(21) Application number: 11846065.8
(22) Date of filing: 06.12.2011
(51) Int. Cl.: C07C 215/28, C07C 213/00

(54) **NOVEL PROCESS FOR SYNTHESIZING FINGOLIMOD HYDROCHLORIDE**

(30) Priority: 25.01.2011 CN 201110026280
(71) Applicant: Shanghai Growing Chem Co., Ltd., Shanghai 200062 (CN)
(72) Inventor: XIAO, Feng, Putuo District Shanghai 200062 (CN); HU, Feng, Putuo District Shanghai 200062 (CN)
(74) Representative: Tombling, Adrian George
(86) International application number: PCT/CN2011/002034
(87) International publication number: WO 2012/100399

(57) **Abstract**

The invention discloses a new synthetic route of Fingolimod hydrochloride, which includes the following steps: step1, reacting compound (5) with sulfhydryl compounds 1 under alkaline conditions, and obtaining key intermediate compound (6), step2, obtaining sulfone compound 7 through oxidation reaction of compound 6, step3, obtaining key intermediate compound (9) through Knoevenagel condensation reaction of the sulfone compound (7) and raw materials (aldehyde compound 8) under alkaline conditions, step4, obtaining 5- amino - 5 - [2 - (4 - N-octyl phenyl) ethyl ] - 2,2- two methyl - 1,3- two oxygen six ring through hydrogenation reduction reaction and deaminate protection of compound (9); then removing acetonide protection in the dilute hydrochloric acid solution, obtaining hydrochloride at the same time, hence getting the target compound (1) of Fingolimod hydrochloride.

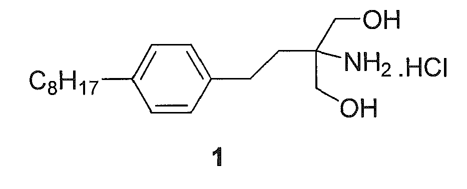

The advantage of the route of synthetic are as follows: the raw materials are cheap and easily obtained, and are easily to treat after reaction. It is not only to the safety of the experiment and high yield but also to reduce the pollution of the environment.

## Description

### Field of the technology

The present invention relates to a new synthetic route of Fingolimod hydrochloride (FTY720) on treatment of multiple sclerosis

### Background of the technology

Multiple sclerosis (multiple sclerosis, MS) is a disease of the central nervous demyelinating system. It's always seen in the young and middle-aged people. Lesions are disseminated widely.

Remission of recurrent neurological lesions symptom often occurs in the course of the disease. The disease causes pathological changes in the brain or spinal cord. Nerve cells have many branched nerve fibers, which are like perplexing as the wire. Multiple sclerosis produces symptoms because of different sizes of the massive demyelination in the central nervous system. The so-called" hardening" refers to these demyelinating areas for tissue repair process resulting in scar tissue and hardening. These lumps may be several, as time progresses, the new lump will appear possibly, then called " multiple".

Multiple sclerosis is a peculiar disease of the nervous system, and it always occurs in the young and middle-aged people with 20~ 40 years old. However, the etiology of multiple sclerosis is still unknown. There are many different theories about why myelin lesions can be produced, and it is common to be considered as an autoimmune disease, that is to say the immune system mistakes myelin as foreign substances to destroy. The personal physique or virus may have contributed to this immune response; some evidence prove that: it was caused by chronic HBV infection, the insulating sheath nerve spinal cord damage, leading to cerebral and spinal nerve bundles of nerve conduction error.

Although the drugs which can be radical to multiple sclerosis is not found yet, there are many treatment routes to control the disease and the sequela from disease. Symptoms caused by the diseases such as stiffness, cramps, pain, stool function disorder, combined with medication and rehabilitation treatment can make the symptom improvement. Corticosteroids may be used in the treatment of acute seizures. It was reported that β-interferon can reduce the frequency of recurrence and relapse severity.

FTY720, whose chemical name is 2 - amino - 2 - [2 - (4 - octylphenyl ) ethyl -1] - 1,3 -diol hydrochloride, is commonly called Fingolimod hydrochloride, and the English name is 2-amino-2- [2 - (4-octylphenyl ) ethy-1] - 1,3 -diol hydrochloride. The structural formula is as follows, 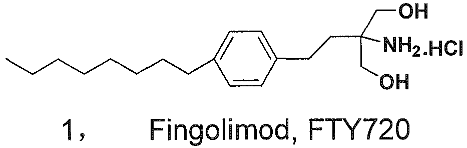 FTY720 is made by structural modification of immunosuppressive effects of component ISP-1 from the Cordyceps. FTY720, which is a new synthetic immunosuppressants, could selectively affect on the lymphocyte, and be mediated by apoptosis or homing, so as to reduce the number of peripheral lymphocytes, but there is no effect on granulocyte and monocyte activity, and it is a low toxicity, high selective immunosuppressive preparation. FTY720 has completely different mechanisms of immune suppression, which can change the orientation of lymphocyte, and also can change the synthesis and expression of intercellular adhesion molecule 21, and could induce apoptosis of lymphocytes by changing the Bc122 / Bax ratio. FTY720 has a good synergistic effect with a variety of immune inhibitor ( CsA, FK506), containing high bioavailability and low adverse drug reaction, and great prospects of clinical application. FTY720 only affects certain types of cells, thus, it has enormous advantages on the treatment for MS, which has been approved for the treatment of MS by the European Union and many other organizations and countries.

There are many publicly reports about synthetic routes of Fingolimod hydrochloride, such as: Patent of the United States :US5609226. Patent of China: CN1528738. Periodical « synthesis » Synthesis, 2000( 4) : 505-506 ) , « Journal of medicinal chemistry» (J Med Chem., 2000, 43(15); Synlet. 2001 (9), 1411-1414); «The Journal of new drugs of China», 2006, 802 and so on. There are some main disadvantages which was used in the prior synthetic route in the open literature as follows,

The synthetic route was long, which needed Fu G reaction, post processing is difficulty, and pollution is serious.

The cost of raw materials of a reducing agent LiAIH4 is too high when is in the synthesis of certain intermediate, and it have great potential safety hazard in the production of LiAIH4.

Some intermediate 2 - (4 - octyl phenyl) Ethyl chloride in some route is instable and not easy to store , and it is difficult to meet the growing market demand.

The key of synthetic steps is that, there commonly exists low yield and difficult treatment of two splicing fragment reaction.

### Summary of the Invention

The present invention is to provide a new synthetic route of Fingolimod hydrochloride. The route does not require the severe reaction conditions and higher cost of reagents such as titanium tetrachloride, lithium aluminium hydride, lithium hydroxide and so on. The raw materials are cheap and easily obtained, and are easily to treat after reaction. It is not only to the safety of the experiment and high yield but also to reduce the pollution of the environment.

To achieve the above-mentioned purposes, the present invention provides a new synthetic route of Fingolimod hydrochloride, which is shown as follows, 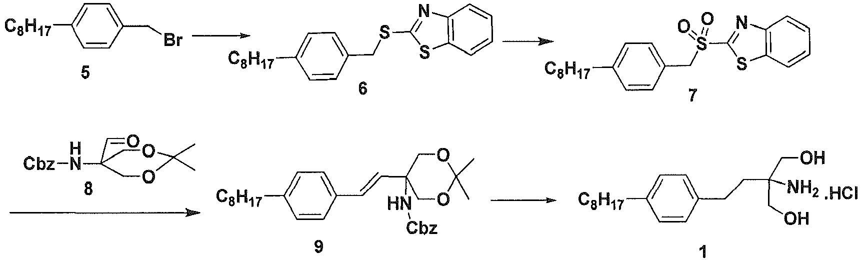
Step 1, reacting compound 5 with 2-mercapto benzothiazole under alkaline conditions, and obtaining key intermediate compound 6;
Step 2, obtaining sulfone compound 7 through an oxidation reaction of compound 6;
Step 3, obtaining key intermediate compound 9 through a Knoevenagel condensation reaction of the sulfone compound 7 and aldehyde compound (8) under alkaline conditions; and
Step 4, obtaining 5- amino - 5 - [2 - (4 - N-octyl phenyl) ethyl] - 2,2- two methyl - 1,3-two oxygen six-membered ring through a hydrogenation reduction reaction and an amino deprotection of compound 9; then removing acetonide protection in dilute hydrochloric acid solution, and obtaining the hydrochloride salt at the same time, hence obtaining the target compound (1) of Fingolimod hydrochloride.

In step 1, the synthetic route of the compound 5 can be as follows, 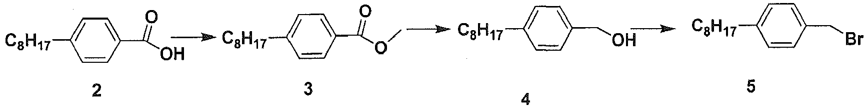 using 4-N-octyl phenyl formic acid 2 as starting material, obtaining product 3 through conventional esterification reaction, then reduction to get product 4, and finally through bromination reaction to get said compound 5. The starting materials are cheap and easy to obtain. The route can be extremely high yield of the compound 5 through conventional esterification and reduction and bromination reaction.

In step 2, the oxidation reaction is completed by adding an oxidizer. The oxidizer is selected from any one of H2O2, chloroperbenzoic acid, and potassium monopersulfate compound salt. The preferred oxidizer is chloroperbenzoic acid.

In step 3, the molar ratio of sulfone compound (7) and aldehyde compound (8) is 1:1.1~1.5 in Knoevenagel condensation reaction. The alkaline is any of potassium carbonate, NaOH and potassium tert-butoxide (t-BuOK). The solvent can be any of ethanol, four tetrahydrofuran, DMF, or their mixture. The preferred one is the mixed solvent of four tetrahydrofuran and DMF.

In step 4, the conditions of said hydrogen reduction reaction are such that the hydrogen pressure is 1 ~ 20kg / cm2, the solvent is an alcohol, specifically, is preferably C1 ~ C4 of lower monohydric alcohol or dihydric alcohol; and the catalyst is carbon on palladium with a weight percentage of 5%-10%.

The invention adopts a new synthetic route, uses the easily obtained starting raw materials, to get the compound 5 by high yield through conventional esterification reaction, reduction reaction, bromination reaction; and then to obtain key intermediates compound 9 through sulfhydryl substitution reaction, oxidation reaction, Knoevenagel condensation reaction, finally, to obtain the target compound Fingolimod hydrochloride through reduction reaction, deprotection, and hydrochloride producing. The invention uses the easily obtained starting raw materials, avoids the use of titanium tetrachloride, lithium aluminium hydride, lithium hydroxide and other reagents, with the mild chemical reaction conditions, convenient operation, high yield, low cost, small environmental pollution. It has a good value in industrial production.

### Detailed Description of the Embodiments

The following will be the embodiments further describe the invention. The embodiments are merely illustrative of the invention rather than the limit scope of the present invention.

### The synthesis of compound 6

### Example 1

4 -octyl benzyl bromide (10.0g, 35.5mmol) , 2 - mercapto benzothiazole (6.5g, 39 mmol) and potassium carbonate (5.4g, 39 mmol) are dissolved in DMF (100ml). The mixture is heated to reflux for 3h. After completion of the reaction, water (100ml) is added to the mixture. Then the mixture is extracted with EA (ethyl acetate). The organic phase are combined and washed with water and brine solution, then dried with Na2SO4. The solution is concentrated under vacuum to give the compound 6 (13.0g) which is pale yellow solid. The yield is 99%.

The melting point data and NMR data of this compound 6 are as follows: Mp:52-54°C;
1H NMR (500HMz, CDCl3):δ=0.87 (t, 3 H), 1.26-1.29 (m, 10 H), 1.57-1.59 (m, 2 H), 2.57 (t, 2 H), 4.58 (s, 2 H), 7.14 (d, J = 8 Hz, 2 H), 7.25-7.31 (m, 1 H), 7.35 (d, J = 8 Hz, 2 H), 7.40-7.43 (m, 1 H), 7.74 (d, J = 8 Hz, 1 H) , 7.89 (d, J = 8 Hz, 1 H).

### 2. The synthesis of compounds 7

### Example 2

The compound 6 (7.4g , 20mmol) is dissolved in chloroform (150ml). M-Chloroperbenzoic acid (14.8g,42mmol) is added to the solution in three equal portions with ice bath. The mixture is stirred at room temperature for 4h. After completion of the reaction, the mixture is washed with sodium bicarbonate solution, sodium sulfite solution, water and brine solution in sequence. Then the solution is dried with Na2SO4, and concentrated under vacuum. The residual pale yellow solid is recrystallized in ethyl acetate, and the product is the compound 7 (6.8g). The yield is 85%.

### Example 3

The compound 6 (7.4g, 20mmol) is dissolved in acetic acid(30ml). To this solution 30% hydrogen peroxide(5.0g, 42mmol) is added at 0°C. Then the solution is stirred at room temperature for 20h. After completion of the reaction, the solution is poured into water (100ml). The mixture is extracted with ethyl acetate(30ml*3). The organic layers are combined and washed with sodium bicarbonate solution, sodium sulfite solution, water and brine solution in sequence. Then the solution is dried with Na2SO4, and concentrated under vacuum, the residual pale yellow solid is recrystallized in ethyl acetate to give compound 7(6.0g). The yield is 75%.

### Example 4

The compound 6 (7.4g, 20mmol) is dissolved in methanol-water solution (80%, 80ml). To the solution Oxone (4eq) is added in four equal portions at 0°C. Then the mixture is stirred at room temperature for 20h. After completion of the reaction, the mixture is filtered. The filtrate is concentrated under vacuum. The residue is crystallized at 0°C and filtered to get crude product which was yellow solid. Then the crude product is recrystallized in EA to give compounds 7(6.2g). The yield is 77%.

The melting point data and NMR data of this compound 7 are as follows:
Mp:75-76 °C ;
1H NMR (500HMz, CDCl3): δ=0.86 (t, 3 H), 1.24-1.30 (m, 10 H), 1.52-1.55 (m, 2 H), 2.53 (t, 2 H), 4.71 (s, 2 H), 7.07 (d, J = 8 Hz, 2 H), 7.15 (d, J = 8 Hz, 2 H), 7.55-7.58 (m, 1 H), 7.62-7.65 (m, 1 H), 7.92 (d, J = 8 Hz, 1 H) , 8.25 (d, J = 8 Hz, 1 H).

### 3. The synthesis of compounds 9

### Example 5

Compound 7 (13g, 32mmol), compound 8 (14g, 48mmol) and anhydrous potassium carbonate (13g, 94mmol) are suspended in THF(100ml) and DMF(30ml), the mixture is heated to reflux for 6h. After completion of the reaction and natural cooling, water (600ml) is added, then extracted with ethyl acetate. The organic phase are combined and washed with sodium carbonate solution, water and brine solution in sequence. Then the solution is dried with Na2SO4, and concentrated under vacuum. The residual pale yellow solid is recrystallized in PE (petroleum ether) to give compounds 9(13g). The yield is 84%.

### Example 6

Compound 7 (1.3g, 3.2mmol) , compound 8 (1.1g, 3.8mmol) and anhydrous potassium carbonate (0.3g, 7.5mmol) are suspended in methanol-water solution with concentration of 80%(20ml). The solution is heated to reflux overnight. After completion of the reaction and self-cooling, water (100ml) is added before extracted with ethyl acetate. The organic phase are combined and washed orderly with sodium carbonate solution, water and brine solution. Then the solution is dried with Na2SO4, and concentrated under vacuum. The residual pale yellow solid is recrystallized in PE, and the product was the compounds 9 (1.2g) . The yield is 77%.

### Example 7

Compound 7 (1.3g, 3.2mmol) , compound 8 (1.1g, 3.8mmol) and potassium tert-butoxide (0.9g, 8mmol) are suspended in DMF (10ml). The solution is heated to 90 °C overnight. After completion of the reaction and natural cooling, water (50ml) is added and extracted with ethyl acetate. The organic phase are combined and washed orderly with sodium carbonate solution, water and brine solution. Then the solution is dried with Na2SO4, and concentrated under vacuum. The residual pale yellow solid is recrystallized in PE to give product (1.0g) which is the compounds 9. The yield is 60%.

### Example 8

Compound 7 (1.3g, 3.2mmol) , compound 8 (1.1g, 3.8mmol) and anhydrous potassium carbonate (0.3g, 7.5mmol) are suspended in methanol-water solution (80%, 20ml). The solution is heated to reflux overnight. After completion of the reaction and self-cooling, water (100ml) is added and extracted with ethyl acetate. The organic phase are combined and washed with sodium carbonate solution, water and brine solution in sequence. Then the solution is dried with Na2SO4, and concentrated under vacuum. The residual pale yellow solid is recrystallized in PE:EA=10:1 to give the product of 5-N-Cbz-5- [2- (4- octyloxyphenyl) diethenyl]- 2,2-dimethyl-1,3- dioxane (1.2g), which is the compounds 9. The yield is 77%.

The melting point data and NMR data of this compound 9 are as follows:
Mp:70-72 °C;
1H NMR (500HMz, CDCl3): δ=0.89 (t, 3 H), 1.28-1.31 (m, 10 H), 1.47-1.50 (m, 6 H), 1.58-1.61 (m, 2 H), 2.59 (t, 2 H), 3.92-3.95 (m, 2 H), 4.01-4.03 (m, 2 H), 5.12 (s, 2 H), 5.54 (s, 1 H), 6.18 (d, J = 16 Hz, 1 H), 6.51 (d, J = 16 Hz, 1 H), 7.12-7.13 (m, 2 H),
7.26-7.28 (m, 2 H), 7.33-7.37 (m, 4 H).

### 4. Synthetic route of Fingolimod hydrochloride(1), that is, 2 - amino - 2 - [2 - (4 - octylphenyl) ethyl -1] - 1,3 -diol hydrochloride,

### Example 9

5-N-Cbz-5- [2- (4- octyloxyphenyl) diethenyl]-2,2 -dimethyl-1,3 -dioxane (2g , 0.0043mol) and 10% Pd/C (0.2g) are added in absolute methanol (20ml), after that hydrogen is introduced and reacted for 2h at room temperature. When the reaction is completed, the mixture is filtered under vacuum to eliminate the Pd/C, and give the intermediate product, namely 5-azyl-5- [2- (4- octyloxyphenyl) diethenyl]-2,2 -dimethyl-1,3 -dioxane. The intermediate product (0.7g, 0.0021 mol) is added directly to 1 N HCl (2.5ml) , then the solution is heated to 50~60°C and reacted for 3h. After completion of the reaction and the cooling to room temperature, the PH is adjusted to alkaline by the addition of saturated sodium carbonate solution. Then EA (10ml) is added to the solution and stirred for 30min to get the white solid educt. The crude product (0.6g) is obtained by vacuum filtration of the mixture, then recrystallized (in EA: Methanol=5:1) to give Fingolimod hydrochloride(0.5g) which is white solid too. The yield is 81%.

The melting point data and NMR data of Fingolimod hydrochloride(1) are as follows: Mp:109-110°C;
1H NMR(500HMz,DMSO): δ=0.85(t,3H), 1.25~1.28(m,10H), 1.52(t, 2H), 1.64~ 1.68(m,2H), 1.97(t,2H), 2.51 ~ 2.53(m,4H),3.42 ~ 3.45 (m,4H), 5.09 (s,2H), 6.38(s,2H),7.08(d,4H)
13C NMR(100HMz,DMSO): δ=13.8,22.0,28.0,28.5,28.7,30.9,31.2,34.2,34.6, 38.8, 58.7,62.2,127.9,128.1,139.1,139.6

Although the content of the present invention has been described in detail with respect to the preferred examples, but it should not be suppose to limit of the invention. It will be obviously that multiple modifications and alternatives for the invention that may occur to one skilled after read the above content. Therefore, the scope of the invention should be limited by the appended claims.

## Claims

1. A method of synthesising Fingolimod hydrochloride (1), wherein said synthetic method comprises the following steps: 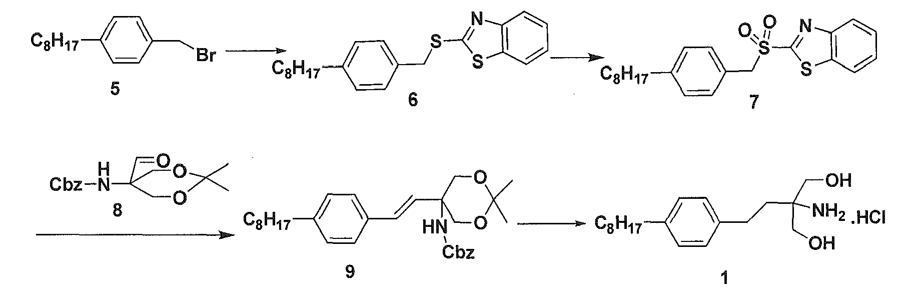
step 1, reacting compound (5) with 2-mercapto benzothiazole under alkaline conditions, and obtaining key intermediate compound (6);
step 2, obtaining sulfone compound (7) through an oxidation reaction of compound (6),
step 3, obtaining key intermediate compound (9) through a Knoevenagel condensation reaction of the sulfone compound (7) and aldehyde compound (8) under alkaline conditions; and
step 4, obtaining 5- amino - 5 - [2 - (4 - N-octyl phenyl) ethyl] - 2,2- two methyl - 1,3-two oxygen six-membered ring through a hydrogenation reduction reaction and an amino deprotection of compound (9); then removing acetonide protection in dilute hydrochloric acid solution, and obtaining the hydrochloride salt at the same time, hence obtaining the target compound (1) of Fingolimod hydrochloride.

2. The method of claim 1, wherein compound (5) is obtained as follows: using 4-N-octyl phenyl formic acid (2) as starting material, obtaining product (3) through conventional esterification reaction, then reduction to get product (4), and finally through bromination reaction to get said compound (5),
the synthetic route of said compound (5) is as follows:

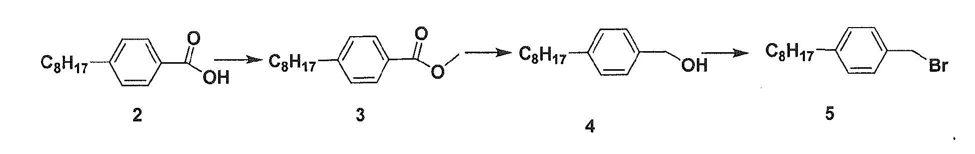

3. The method of claim 1, wherein, in step 2, said oxidation reaction is completed by adding an oxidizer, and the oxidizer is selected from any of H₂O₂, chloroperbenzoic acid, potassium monopersulfate compound salt.

4. The method of claim 1, wherein, in step 3, the molar ratio of sulfone compound (7) and aldehyde compound (8) is 1:1.1~1.5, and wherein the alkaline conditions are produced by any of potassium carbonate, NaOH and potassium tert-butoxide (t-BuOK).

5. The method of claim 1, wherein, in step 4, the conditions of said hydrogen reduction reaction are such that the hydrogen pressure is 1 ~ 20kg / cm², the solvent is an alcohol, and the catalyst is carbon on palladium with a weight percentage of 5%-10%.

6. An intermediate compound of chemical formula 6:

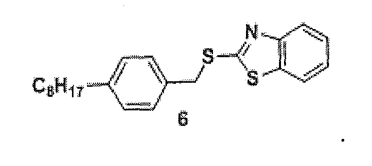

7. An intermediate compound of chemical formula 7:

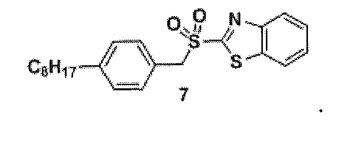

8. An intermediate compound of chemical formula 9:
